Europäisches Patentamt

European Patent Office

Office européen des brevets

(19)

(11) Veröffentlichungsnummer: **0 001 242**
**B1**

(12) **EUROPÄISCHE PATENTSCHRIFT**

(21) Anmeldenummer: **78100843.8**

(22) Anmeldetag: **07.09.78**

(51) Int. Cl³: **C 07 C 69/44,**
**C 07 C 69/52, C 07 C 67/38**

(54) Verfahren zur Herstellung von Butandicarbonsäureestern

(30) Priorität: **15.09.77 DE 2741511**

(43) Veröffentlichungstag der Anmeldung:
**04.04.79 Patentblatt 79/07**

(45) Bekanntmachung des Hinweises auf die Patenterteilung:
**23.07.80 Patentblatt 80/15**

(84) Benannte Vertragsstaaten:
**BE DE FR GB NL**

(73) Patentinhaber: **BASF Aktiengesellschaft**
**Carl-Bosch-Strasse 38**
**D - 6700 Ludwigshafen (DE)**

(72) Erfinder: **Kummer, Rudolf, Dr.**
**Kreuzstrasse 6**
**D - 6710 Frankenthal 5 (DE)**
**Schneider, Heinz-Walter, Dr.**
**Bruesseler Ring 43**
**D - 6700 Ludwigshafen (DE)**
**Weiss, Franz-Josef, Dr.**
**Schlehdornweg 69**
**D - 6940 Weinheim (DE)**

Courier Press, Leamington Spa, England.

Verfahren zur Herstellung von Butandicarbonsäureestern

Gegenstand der Erfindung ist ein Verfahren zur Herstellung von Butandicarbonsäureestern, bei dem man Butadien oder Butadien enthaltende Kohlenwasserstoffgemische mit Kohlenmonoxid und niederen Alkanolen in Gegenwart von tertiären Stickstoffbasen und Kobaltcarbonyl bei Temperaturen von 80 bis 150°C unter erhöhtem Druck umsetzt, und den so erhaltenen Pentensäureester mit Kohlenmonoxid und niederen Alkanolen bei Temperaturen von 140 bis 200°C unter erhöhtem Druck weiter zu Butandicarbonsäureestern umsetzt.

Aus Bull. Chem. Soc. Japan *46*, Seite 524 ff (1973) ist ein zweistufiges Verfahren zur Herstellung von Adipinsäureestern aus Butadien bekannt, wobei man Butadien zunächst mit Kohlenmonoxid und Alkanolen in Gegenwart von Kobaltcarbonyl und Stickstoffbasen wie Pyridin oder Isochinolin umsetzt und, ohne den Katalysator zu entfernen, in einer darauffolgenden Stufe den entstandenen Pentensäureester mit Kohlenmonoxid und Alkanolen weiter zu Adipinsäureester umsetzt. Bei der technischen Durchführung eines solchen Verfahrens ist es jedoch erforderlich, den Katalysator wiederzugewinnen und zurückzuführen. So wurde in dem aus der US—PS 3 778 466 bekanntgewordenen Verfahren der den Katalysator enthaltende Rückstand nach Abdestillieren der Wertprodukte wieder für die Carbonylierung verwendet. Es hat sich jedoch gezeigt, daß beispielsweise nach 4-maliger Verwendung die Wirksamkeit des Katalysators erheblich nachläßt. Dies ist darauf zurückzuführen, daß einerseits durch die Destillation der Katalysator geschädigt wird, da Kobaltcarbonylkomplexe thermisch nicht stabil sind und andererseits bei der Carbonylierung Nebenprodukte entstehen, die die Carbonylierung beeinflussen und deshalb fortlaufend ausgeschleust werden müssen. Es wurde auch schon versucht, nach der Carbonylierung die Wertprodukte durch Extraktion vom Katalysator haltigen Rückstand abzutrennen. So wird in der DE—OS 2 159 139 ein Verfahren beschrieben, bei dem man das Methanol enthaltende Carbonylierungsgemisch mit Kohlenwasserstoffen extrahiert. Hierbei gelingt es zwar ohne Schädigung des Katalysators, die Wertprodukte abzutrennen und die methanolische Katalysator enthaltende Lösung in die Carbonylierung zurückzuführen. Diese extraktive Trennung ist jedoch nicht geeignet, um Nebenprodukte wie polymere Butadiene, die bei der Carbonylierung entstehen, auszuschleusen. Sie reichern sich deshalb bei mehrmaliger Wiederverwendung der Katalysatorlösung fortlaufend an und führen zu Störungen bei der Carbonylierung.

Es war deshalb die technische Aufgabe gestellt, die Carbonylierung von Butadien zu Butandicarbonsäureestern so zu gestalten, daß das Katalysatormetall möglichst vollständig in einer für die Carbonylierung wiederverwendbaren Form zurückgewonnen wird und zugleich die schädlichen Nebenprodukte ausgeschleust werden.

Diese technische Aufgabe wird gelöst in einem Verfahren zur Herstellung von Butandicarbonsäureestern, bei dem man Butadien oder Butadien enthaltende Kohlenwasserstoffgemische mit Kohlenmonoxid und $C_1$- bis $C_4$-Alkanolen in Gegenwart von tertiären Stickstoffbasen und Kobaltcarbonylverbindungen bei Temperaturen von 80 bis 150°C und unter erhöhtem Druck umsetzt und anschließend ohne den Kobaltkatalysator abzutrennen, den erhaltenen Pentensäureester mit Kohlenmonoxid und $C_1$- bis $C_4$-Alkanolen bei Temperaturen von 140 bis 200°C unter erhöhtem Druck weiter zu Butandicarbonsäureestern umsetzt, wobei man.

a) eine wäßrige Kobaltsalzlösung bei Temperaturen von 50 bis 200°C unter Drücken von 50 bis 500 bar mit überschüssigem Kohlenmonoxid und Wasserstoff in Gegenwart von Aktivkohle, die mit Kobaltcarbonyl beladen ist, behandelt,

b) die erhaltene wäßrige Lösung von Kobaltcarbonylwasserstoff mit Butadien oder Butadien enthaltenden Kohlenwasserstoffgemischen extrahiert, die wäßrige Phase abtrennt und,

c) das Kobaltcarbonylhydrid, Kobaltcarbonyl und Butenylkobalttricarbonyl enthaltende Butadien oder solches enthaltende Kohlenwasserstoffgemisch mit Kohlenmonoxid und $C_1$- bis $C_4$-Alkanolen im Überschuß in Gegenwart von 0,5 bis 2 Mol tertiäre Stickstoffbase je Mol Butadien mit einem $PK_a$-Wert von 3 bis 11, bei Temperaturen von 80 bis 150°C unter Drücken von 300 bis 2 000 bar umsetzt,

d) aus dem so erhaltenen Reaktilnsgemisch die darin enthaltenden tertiären Stickstoffbasen bis auf 0,1 bis 0,3 Mol je Mol erzeugten Pentensäureester sowie überschüssige Kohlenwasserstoffe abtrennt, den im Reaktionsgemisch verbleibenden Pentensäureester mit Kohlenmonoxid und $C_1$- bis $C_4$-Alkanolen im Überschuß bei Temperaturen von 140 bis 200°C unter Drücken von 100 bis 400 bar in Gegenwart der im Reaktionsgemisch verbleibenden Menge an Kobaltcarbonyl und tertiärer Stickstoff-base umsetzt und anschließend die überschüssigen Alkanole und freien tertiären Stickstoffbasen abdestilliert,

e) das verbleibende Katalysator, Butandicarbonsäureester und Nebenprodukte enthaltende Reaktionsgemisch mit Oxidationsmitteln in wäßrig saurem Medium behandelt, das Gemisch in eine organische Phase, aus der durch Destillation Butandicarbonsäureester gewonnen werden, und eine Kobaltsalze enthaltende wäßrige Phase trennt mit der Maßgabe, daß man die Kobaltsalze enthaltende wäßrige Phase mit Lösungsmitteln extrahiert, die mit Wasser nicht mischbar sind.

Das neue Verfahren hat den Vorteil, daß aus der wäßrigen Phase zusätzliche Mengen an Wertprodukten gewonnen werden. Abgesehen davon hat die beanspruchte Verfahrensweise den Vorteil, daß mit der wäßrigen Phase keine störenden Produkte im Kreis geführt werden. Die in der wäßrigen Phase enthaltenen $C_5$- und $C_6$-Carbonsäureester verseifen bei mehrfacher Rückführung der wäßrigen Phase. Die hierbei entstehenden Carbonsäuren bilden gemeinsam mit den Kobaltionen und den Stickstoffbasen schwerlösliche Salze, was zu Katalysatorverlusten und Verlegungen in den Leitungen führt. Somit wird durch das neue Verfahren eine fortlaufende Anreicherung solcher Säuren vermieden.

In einer ersten Stufe (Stufe a) werden wäßrige Kobaltsalzlösungen mit Kohlenmonoxid und Wasserstoff im Überschuß bei Temperaturen von 50 bis 200°C und unter Drücken von 50 bis 500 bar in Gegenwart von Aktivkohle, die mit Kobaltcarbonyl beladen ist, behandelt. Bevorzugt verwendet man als Kobaltsalze fettsaure Salze, die wasserlöslich sind, insbesondere Formiate, Acetate, Propionate oder Butyrate. Besonders bewährt haben sich Kobaltformiat und acetate. Zweckmäßig geht man von Lösungen aus, die 0,5 bis 5 Gew.% Kobalt, berechnet als Metall, insbesondere 1 bis 3 Gew.% Kobalt in Form der genannten Salze enthalten. Im allgemeinen enthält das genannte Gasgemisch Kohlenmonoxid und Wasserstoff im Volumenverhältnis 4:1 bis 1:2, insbesondere im Volumenverhältnis 2:1 bis 1:1. Besonders bewährt hat sich ein annähernd äquimolekulares Gemisch aus Kohlenmonoxid und Wasserstoff. Vorteilhaft verwendet man das Gemisch aus Kohlenmonoxid und Wasserstoff in einem Überschuß, z.B. bis zu dem 5-fachen der stöchiometrisch erforderlichen Menge. Vorteilhaft hält man Temperaturen von 100 bis 170°C und Drücke von 100 bis 400 bar ein. Ferner hat es sich als vorteilhaft erwiesen, wenn die wäßrige Kobaltsalzlösung bis zu 20 Gew.%, insbesondere 3 bis 10 Gew.% eines inerten Neutralsalzes, vorzugsweise Alkalisalze nicht oxidierender Säuren wie Alkalisulfate oder Alkaliphosphate enthält.

Die Behandlung in der Stufe a folgt in Gegenwart von Aktivkohle. Geeignete Aktivkohlearten sind z.B. Torfkohle, Tierkohle oder Zuckerkohle. Als besonders geeignet hat sich Torfkohle erwiesen. Die Aktivkohle ist zweckmäßig bis zu deren Sättigung mit Kobaltcarbonyl beladen. Dies wird im allgemeinen dadurch erreicht, daß man wäßrige Lösungen von Kobaltsalzen zusammen mit dem genannten Gasgemisch aus Kohlenmonoxid und Wasserstoff unter den angegebenen Reaktionsbedingungen über die Aktivkohle bis zu deren Sättigung leitet, d.h. bis man im Austrag Kobaltcarbonyl oder Kobaltcarbonylwasserstoff analytisch feststellt.

Im allgemeinen führt man die Behandlung in einer sogenannten Behandlungszone durch, die zweckmäßig ein Verhältnis von Länge zu Durchmesser von 5 bis 50:1 hat, in der die Aktivkohle in der Regel fest angeordnet ist. Vorzugsweise hält man eine Belastung von 1,5 bis 15 g Kobalt, berechnet als Metall, in Form der genannten Salze pro Stunde je Kilogramm Aktivkohle ein.

Die so erhaltene Kobaltcarbonylwasserstoff, nichtumgesetzte Kobaltsalze und freigesetzte Säure enthaltende wäßrige Lösung wird zweckmäßig zusammen mit dem nichtverbrauchten Gemisch aus Kohlenmonoxid und Wasserstoff vorteilhaft ohne Entspannen der zweiten Stufe (Stufe b) zugeführt. Dort wird Kobaltcarbonylwasserstoff mit Butadien oder Butadien enthaltenden Kohlenwasserstoffgemischen, die nachfolgend noch näher erläutert werden, extrahiert. Es ist möglich, die gesamte für die Carbonylierung erforderliche Butadienmenge zur Extraktion zu verwenden oder nur einen Teil derselben. Vorteilhaft verwendet man 5 bis 30 Mol Butadien pro g-Atom zu extrahierendes Kobalt. Die Extraktion wird im Gegenstrom oder Gleichstrom in Vorrichtungen durchgeführt, die in der Technik für die Extraktion eingeführt sind, beispielsweise Kolonnen oder statische Mischer. Während der Extraktion hält man Temperaturen von 20 bis 100°C und Drücke von 5 bis 300 bar ein. Das Gemisch wird anschließend in eine wäßrige Phase und eine organische Phase getrennt. Führt man die Extraktion beispielsweise in einem mit Raschig-Ringen gefüllten Druckrohr durch, so tritt im oberen Teil gleichzeitig eine Trennung in eine organische und ein wäßrige Phase ein. Gleichzeitig wird das mitverwendete Gemisch aus Kohlenmonoxid und Wasserstoff als Gasphase abgetrennt. Der Kobaltgehalt der die Stufe b verlassenden organischen Phase beträgt im allgemeinen von 1 bis 5 Gew.%. Es wird angenommen, daß das Kobaltcarbonyl als Komplexverbindung mit Butadien, die in Wasser unlöslich ist, in der organischen Phase vorliegt.

Die organische Phase wird nun in der Stufe c in Gegenwart von 0,5 bis 2 Mol tertiärer Stickstoffbase je Mol Butadien mit einem $Pk_a$-Wert von 3 bis 11 mit der Maßgabe, daß die tertiäre Stickstoffbase vorzugsweise niedriger sieden soll als der jeweils herzustellende Pentensäureester mit $C_1$- bis $C_4$-Alkanolen im Überschuß bei Temperaturen von 80 bis 150°C und unter Drücken von 300 bis 2 000 bar umgesetzt.

Falls für die Extraktionen nicht die gesamte Menge an Butadien oder Butadien enthaltendes Kohlenwasserstoffgemisch, das für die Carbonylierung benötigt wird, verwendet wurde, setzt man die erforderliche Menge an Ausgangsstoffen in der Stufe c zusätzlich zu. Es sei hier vermerkt, daß anstatt reinem Butadien vorteilhaft Butadien enthantende Kohlenwasserstoffgemisch verwendet werden können. Solche Kohlenwasserstoffgemische enthalten neben Butadien gesättigte Kohlenwasserstoffe mit 3 bis 5 Kohlenstoffatomen und einfach olefinisch ungesättigte Olefine mit 3 bis 5 Kohlenstoffatomen. Der Butadiengehalt soll in der Regel mehr als 10 Gew.% betragen. Der Technik werden insbesondere $C_4$-Schnitte als Ausgangsgemisch verwendet. Als solche seinen alle Gemische von überwiegend unverzweigten $C_4$-Kohlenwasserstoffen definiert, die mehr als 10 Gew.% Butadien-1,3 (Butadien) und mehr als 15% Butene enthalten. Je nach Provenienz liegen die einzelnen Komponenten in solchen Gemischen vormalerweise in folgenden Anteilen vor:

| Butadien | 10 bis 70 durchschnittlich 40 bis 60 Gew.% |
|---|---|
| Isobuten | 15 bis 40 durchschnittlich 20 bis 35 Gew.% |
| But-1-en | 10 bis 40 durchschnittlich 10 bis 25 Gew.% |
| But-2-en | 5 bis 20 durchschnittlich 5 bis 15 Gew.% |
| Butane | 1 bis 10 durchschnittlich 1 bis 10 Gew.% |
| Butine | 0,1 bis 3 durchschnittlich 0,1 bis 3 Gew.% |

Solche $C_4$-Schnitte fallen beispielsweise an bei der Dehydrierung von Butan oder Buten oder als Nebenprodukte bei der Äthylengewinnung durch thermische Spaltung (Cracken) von Leichtbenzin (Naphtha) oder höheren Kohlenwasserstoffschnitten.

Die geeigneten tertiären Stickstoffbasen sind vorzugsweise N-heterocyclische Verbindungen wie Pyridin ($Pk_a$ 5,3), Methylpyridine wie 3-Picolin ($Pk_a$ 6,0), Isochinolin ($Pk_a$ 5,4) ferner Trialkylamine wie Trimethylamin ($Pk_a$ 9,8) oder Triäthylamin ($Pk_a$ 11,0). Besondere technische Bedeutung hat Pyridin erlangt.

Besonders bewährt hat essich 0,6 bis 1,5 Mol tertiäre Stickstoffbasen je Mol Butadien anzuwenden.

Vorzugsweise verwendet man tertiäre Stickstoffbasen, die niedriger sieden als die jeweils herzustellenden Pentensäureester.

Als geeignete $C_1$- bis $C_4$-Alkanole seien genannt Methanol, Äthanol, Propanol, Butanol, Isobutanol. Besonders bevorzugt wird Methanol verwendet.

Die Umsetzung führt man vorzugsweise bei Temperaturen von 120 bis 140°C und Drücken von 600 bis 1 200 bar durch. Je Mol Butadien verwendet man in der Regel 0,01 bis 0,1 g-Atom Kobalt in Form der beschriebenen Kobaltcarbonylkomplexe an.

In diesem Zusammenhang ist es bemerkenswert, daß bei der Verwendung von $C_4$-Gemischen die außerdem vorhandenen Butene unter den angegebenen Reaktionsbedingungen nicht zu den entsprechenden Carbonsäureestern reagieren, obwohl dies gemäß DE—OS 2 023 690 zu erwarten gewesen wäre.

Das so erhaltene Reaktionsgemisch enthält neben nichtumgesetztem Butadien gegebenenfalls andere Kohlenwasserstoffe, tertiäre Stickstoffbasen, Kobaltcarbonyl, nicht umgesetzte Alkanole, die als Wertprodukte erzeugten Pentensäureester sowie Nebenprodukte, wie Valeriansäureester, Vinylcyclohexen, Butenyl- und Butylketone sowie Polymerisate des Butadiens.

Aus dem so erhaltenen Reaktionsgemisch werden nach dem Entspannen die darin enthaltenen tertiären Stickstoffbasen bis auf 0,1 bis 0,3 Mol je Mol Pentensäureester sowie gegebenenfalls überschüssige Kohlenwasserstoffe abgetrennt (Stufe d). Die Abtrennung kann durch Destillation oder andere Trannverfahren wie Extraktion erfolgen. Vorteilhaft werden tert. Stickstoffbasen gegebenenfalls überschüssige Kohlenwasserstoffe durch Destillation unter vermindertem Druck abgetrennt.

Hierbei soll die Temperatur im Destillationssumpf 75°C nicht überschreiten, um eine Zersetzung des Kobaltkatalysators zu vermeiden. Je nach Wahl des mitverwendeten Alkanoles destilliert gleichzeitig auch ein Teil oder das gesamte überschüssige Alkanol ab.

Der im Reaktionsgemisch verbleibende Pentensäureester wird mit Kohlenmonoxid und $C_1$- bis $C_4$-Alkanolen im Überschuß, nachdem gegebenenfalls eine entsprechende Menge Alkanole erneut zugegeben wirde, bei Temperaturen von 140 bis 200°C und unter Drücken von 100 bis 400 bar in Gegenwart der im Reaktionsgemisch vorhandenen Menge an Kobaltkatalysator und tertiärer Stickstoffbase umgesetzt. Vorteilhaft hält man Temperaturen von 150 bis 180°C und Drücke von 100 bis 400 bar ein. Vorzugsweise beträgt die mitverwendete Menge an Alkanolen 1,5 bis 4 Mol je Mol Pentensäureester. Es hat sich ferner als vorteilhaft erwiesen, dem Kohlenmonoxid einige Volumenprozent Wasserstoff, z.B. 1 bis 4 Volumenprozent zuzugeben, um die Reaktionsgeschwindigkeit zu erhöhen. Nach dem Entspannen werden aus dem erhaltenen Reaktionsgemisch das überschüssige Alkanol und die freie tertiäre Stickstoffbase abdestilliert. Die chemisch gebundene tertiäre Stickstoffbase (1 bis 2 Mol pro g-Atom Kobalt) wird dabei nicht abdestilliert. Um eine Zersetzung des Kobalt-Komplexes unter Ausscheidung von Kobaltmetall zu vermeiden, die unerwünscht ist, hat es sich als zweckmäßig erwiesen, in den Sumpf der Kolonne einen langsamen Strom von Kohlenmonoxid oder Kohlenmonoxid enthaltenden Gasen einzuleiten.

Das verbleibende Katalysator, Butandicarbonsäureester und Nebenprodukte enthaltende Reaktionsgemisch wird in Stufe e mit Oxidationsmitteln in wäßrig-saurem Medium behandelt. Als Oxidationsmittel sind insbesondere solche geeignet, die das Reaktionsgemisch nicht verunreinigen, beispielsweise Wasserstoffperoxid, Sauerstoff oder Sauerstoff enthaltende Gase. Besonders bevorzugt werden molekularen Sauerstoff enthaltende Gase, insbesondere Luft, verwendet. Das Oxidationsmittel wird mindestens in einer Menge von zwei Oxidationsäquivalenten je Mol Kobaltverbindung verwendet. Vorteilhaft wendet man jedoch einen Überschuß an. In der Praxis hat es sich als zweckmäßig erwiesen, 30 bis 300 Nl Luft je kg Reaktionsgemisch anzuwenden.

Im allgemeinen verwendet man die 0,1- bis 10-fache, vorteilhaft 0,2- bis 1-fache Gewichtsmenge Wasser, bezogen auf das Reaktionsgemisch. Der pH-Wert beträgt vorteilhaft zwischen 3 und 6. Als Säuren eignen sich nichtoxidierende Mineralsäuren oder Fettsäuren. Besonders geeignet ist die in Stufe b anfallende wäßrige sauren Lösung nach Abtrennung des Kobaltcarbonylwasserstoff enthaltenden Butadiens.

Eine solche Lösung enthält z.B., falls man von Kobaltacetat ausgeht, neben nichtumgesetzem Kobaltacetat Essigsäure. Falls es erforderlich ist, kann zusätzlich eine geeignete Fettsäure zugegeben werden. Auf jeden Fall ist darauf zu achten, daß genügend Säure vorhanden sit, um das Kobaltsalz in Lösung zu halten. Das gleiche gilt für die anzuwendende Wassermenge. Um die Kobaltlösung nicht in zu großer Verdünnung zu erhalten, ist es zweckmäßig, die wäßrige kobalthaltige Lösung im Kreis in den Behandlungsraum zurückzuführen und nur einen kleinen Teilstrom, der der zugeführten Menge entspricht, abzutrennen.

Die Behandlung erfolgt vorteilhaft bei Temperaturen von 80 bis 160°C. Besonders bewährt haben sich Temperaturen von 100 bis 130°C. Je nach Grad der Durchmischung ist die Umsetzung bereits nach wenigen Sekunden vielfach bereits innerhalb von Bruchteilen einer Sekunde beendet. Um eine gute Durchmischung zu gewährleisten ist es zweckmäßig, das Reaktionsgemisch unter gleichzeitiger Zuführung des Oxidationsmittels, z.B. Luft, in feiner Verteilung der wäßrig-sauren Lösung zuzuführen.

Nach der Behandlung trennt man die Flüssigphase, z.B. durch Dekantieren, in eine organische Phase und eine wäßrige Phase. Um die Phasentrennung zu erleichtern hat es sich als vorteilhaft erwiesen, wenn das wäßrig-saure Milieu inerte Neutralsalze, z.B. Alkalisalze von nichtoxidierenden Säuren wie Natriumsulfat in Mengen bis zu 20% enthält. Ferner ist es vorteilhaft, die organische Phase in einer Menge bis zu 20 Gew.% mit Kohlenwasserstoffen zu versetzen. Hierzu eignen sich insbesondere die auch in der Stufe d nach der ersten Carbonylierung abdestillierten überschüssigen Kohlenwasserstoffe.

Erfindungsgemäß wird die in der Stufe e anfallende Kobaltsalz enthaltende wäßrige Phase mit Lösungsmitteln extrahiert, die mit Wasser nicht mischbar sind.

Als Extraktionsmittel sind sämtliche Lösungsmittel, die unter den Extraktionsbedingungen inert sind und die mit Wasser nicht mischbar sind, geeignet. Bevorzugt verwendet man Kohlenwasserstoffe wie Hexan, Cyclohexan, Pentan oder $C_4$-Schnitt (Gemische aus Butan und Butenen), ferner Ketone wie Dibutylketon, Diisopropylketon oder Methylisobutylketon. Andere geeignete Lösungsmittel sind Äther wie Diäthyläther, Diisopropyläther oder Methylisobutyläther. Besonders bevorzugt werden kW als Extraktionsmittel verwendet.

Die Extraktion läßt sich in allen dafür geeigneten in der Technik eingeführten Vorrichtungen durchführen. Beispielsweise sind geeignet: Pulsationskolonnen, statische Mischer oder nach dem sogenannten Mixer-Settler-Prinzip arbeitende Vorrichtungen. Vorteilhaft führt man die Extraktion im Gegenstrom durch. In der Regel wird die Extraktion bei Temperaturen durchgeführt, bei denen die wäßrige Phase nach der Aberennung der organischen Phase anfällt, z.B. bei 40 bis 80°C.

Nach Abtrennen der organischen Phase wird die Kobaltsalze enthaltende wäßrige Phase wieder zur Herstellung der Katalysatorlösung, z.B. in der Stufe a, verwendet. Es hat sich ferner als vorteilhaft erwiesen, die anfallende organische Phase dem Reaktionsgemisch nach der oxidativen Behandlung im wäßrig-sauren Milieu gemäß Stufe e zuzuführen. Hierdurch wird eine bessere Trennung in eine organische und eine wäßrige Phase erreicht. Das organische Lösungsmittel wird anschließend bei der Gewinnung der Butandicarbonester durch Destillation wiedergewonnen und erneut zur Extraktion der wäßrigen Phase eingesetzt.

In der Regel verwendet man je Volumenteil Kobaltsalze enthaltende wäßrige Lösung 0,5 bis 2 Teile Lösungsmittel.

Es hat sich ferner als vorteilhaft erwiesen, wenn man die im Kreis geführte wäßrige Kobaltsalze enthaltende wäßrige Lösung mit stark basischen Ionenaustauschern, vorzugsweise in der Acetatform, behandelt. Geeignete Ionenaustauscher sind beispielsweise solche auf Basis vernetzter Polystyrole, die quaternäre Ammoniumgruppen enthalten. Der vierte Ligand am N-Atom ist hierbei vorzugsweise ein Methyl-, Hydroxyäthyl- oder Benzylrest. Die Kapazität der Ionenaustauscher beträgt im allgemeinen 0,5 bis 3 Val./1.

Solche Ionenaustauscher sind z.B. bekannt als Amberlite® IRA 400 der Firma Rohm and Haas Philadephia.

Die Behandlung mit Ionenaustauschern führt man beispielsweise nach der Extraktion der Kobaltcarbonylwasserstoff enthaltenden wäßrigen Phase durch. Vorteilhaft nimmt man die Behandlung also vor an der wäßrigen Phase, die in der Stufe b abgetrennt wird, bevor diese in die Stufe e zurückgeführt wird. Es ist möglich, die gesamte wäßrige Phase von Zeit zu Zeit, z.B. wenn der Gehalt an Adipinsäure 1,5 Gewichtssprozent erreicht hat, zu behandeln oder fortleufend mit einem Teilstrom die Behandlung durchzuführen.

Aus der organischen Phase erhält man durch fraktionierte Destillation restliches Pyridin, nichtumgesetzten Pentensäureester, die wieder der Carbonylierung zugeführt werden, sowie ein Gemisch aus Butandicarbonsäureestern (80 bis 85 Gew.% Adipinsäureester, 11 bis 15 Gew.% 2-Methylglutarsäureester und 3 bis 6 Gew.% 2-Äthylbernsteinsäureester). Das Estergemisch kann zur Herstellung von Diolen oder Polyestern verwendet werden. Der aus dem Estergemisch durch fraktionierte Destillation erhältliche Adipinsäureester eignet sich zur Herstellung von Adipinsäure, AH-Salz, Adipodinitril und Hexandiol-1,6.

Die Kobaltsalze enthaltende wäßrige Phase wird vorteilhaft wieder in die Stufe a als Ausgangslösung zur Herstellung von Kobaltcarbonylwasserstoff zurückgeführt. Das Verfahren nach der Erfindung eignet sich hervorragenderweise für eine kontinuierliche technische Durchführung.

Butandicarbonsäureester eignen sich zur Herstellung von Polymeren.

Das Verfahren nach der Erfindung sei an folgenden Beispielen veranschaulicht.

*Beispiel*

Ein Hochdruckrohr, das mit 600 ml Aktivkohle (Firma Norit, Körnung 3 bis 5 mm) gefüllt ist, wird stündlich mit 180 ml einer wäßrigen Kobaltacetatlösung, die 2,5 Gewichtsprozent Kobalt$^{2+}$ enthält, beschickt. Die wäßrige Kobaltacetatlösung fällt in der Stufe e als wäßrige Phase an. Außerdem führt man stündlich 50 Normalliter eines äquimolaren Gemisches aus Kohlenmonoxid in Wasserstoff zu. Hierbei hält man eine Temperatur von 120°C und einen Druck von 300 bar ein. Die im unteren Teil des Rohres abgezogene Lösung enthält 0,65 Gewichtsprozent Kobalt$^{2+}$ und 1,85 Gewichtsprozent Kobalt als Kobaltcarbonylwasserstoff, ferner die entsprechende Menge an Essigsäure. Diese Lösung wird nach dem Entspannen auf 20°C bei Raumtemperatur intensiv mit 310 ml eines C$_4$-Schnittes, der 43 Gewichtsprozent Butadien (1,57 Mol) enthält, gemischt. Nach der Phasentrennung enthält der C$_4$-Schnitt 3,7 g Kobalt in Form von Kobaltcarbonylverbindungen Dieser Kobalt enthaltende C$_4$-Schnitt wird nun einem Hochdruckgefäß von 1,9 l Inhalt zugeführt und außerdem stündlich 127 ml (1,57 Mol) Pyridin, 127 ml Methanol (3,14 Mol) und 60 Normalliter Kohlenmonoxid zugegeben. Die Carbonylierung verläuft bei einer Temperatur von 130°C und 600 bar. Das im Kopf des Hochdruckgefäßes abgenommene Produkt wird entspannt, wobei gasförmig neben überschüssigem Kohlenmonoxid überschüssige C$_4$-Kohlenwasserstoffe abgetrennt werden. Diese enthalten praktisch kein Butadien. Unter vermindertem Druck, um den Katalysator zu schonen werden von dem Austrag stündlich etwa 52 g Methanol und 100 g Pyridin abdestilliert. Im Destillationssumpf hält man eine maximale Temperatur von 65°C ein. Dieser Destillationssumpf, der 3,7 g Kobalt als Carbonylkomplex 165 g (1,44 Mol) Pentensäureester enthält, wird zusammen mit 117 ml (2,88 Mol) Methanol und 55 Normalliter Kohlenmonoxid, das 2 Volumenprozent Wasserstoff enthält, einem weiteren Hochdruckgefäß von 1,7 Liter Inhalt kontinuierlich von unten zugeführt. Die Carbonylierung wird bei einer Temperatur von 170°C und unter einem Druck von 150 bar durchgeführt. Aus dem Austrag wird in einer weiteren Kolonne unter Einleiten von etwa 50 Normalliter Kohlenmonoxid pro Stunde das überschüssige Methanol und das freie Pyridin abdestilliert. Der Destillationssumpf (265 g pro Stunde) wird mit 200 ml pro Stunde der in der Extraktionsstufe b anfallenden essigsa uren wäßrigen Lösung unter Durchleiten von etwa 50 Normalliter Luft bei 100°C in einem mit Raschig-Ringen gefüllten Rohr gut durchgemischt.

Nach der Phasentrennung werden 200 ml wäßrige Kobaltacetatlösung erhalten, aus der geringe Mengen Pyridin abdestilliert werden. Die organische Phase wird durch fraktionierte Destillation aufgearbeitet. Die erhaltene wäßrige Phase wird mit gleichen Volumenteilen Cyclohexan in einer Gegenstrompulsationskolonne von 1,5 m Länge und 30 mm Durchmesser, die mit Raschigringen gefüllt ist, extrahiert. Die organische Phase wird in die Stufe e vor der Phasentrennung zugeführt. Nach der Extraktion sind in der wäßrigen Phase kein Adipinsäuredimethylester und nur Spuren von Adipinsäuremonomethylester nachweisbar. Die wäßrige Lösung wird wieder als Ausgangslösung für die Katalysatorbildung in die Stufe a zurückgeführt. Nach 25 Kreisläufen der Wasserphase konnte noch kein Ausfallen von schwerlöslichen Kobaltsalzen beobachtet werden. Im weiteren Verlauf wird die wäßrige Phase nach der Extraktion zusätzlich mit einem stark basischen Ionenaustauscher (Amerlite® IRA 400 von Rohm and Haas Philadelphia) in der Acetatform behandelt. In der wäßrigen Lösung kann keine freie Adipinsäure mehr festgestellt werden.

*Vergleichsbeispiel*

Man verfährt wie in Beispiel 1 beschrieben ohne die wäßrige Lösung zu extrahieren oder mit Ionenaustauschern zu behandeln. In der wäßrigen Phase zur Stufe e verbleiben 1,5 Gewichtsprozent Adipinsäuredimethylester. Nach 5 Kreisläufen der Wasserphase hat sich unter Hydrolyse der mitgeführten Ester ein Gehalt von 1,3% Adipinsäuremonomethylester und 1,5% Adipinsäure aufgebaut. Bei der Weiterführung der Raktion kommt es in der wäßrigen Phase zu Ausfällungen von Kobaltpyridinadipaten, was zu Verlusten an Katalysator führt.

## Patentansprüche

1. Verfahren zur Herstellung von Butandicarbonsäureestern, bei dem man Butadien oder Butadien enthaltende Kohlenwasserstoffgemische mit Kohlenmonoxid und $C_1$- bis $C_4$-Alkanolen in Gegenwart von tertiären Stickstoffbasen und Kobaltcarbonyl bei 80 bis 150°C unter erhöhtem Druck umsetzt und ohne den Katalysator abzutrennen dann den erhaltenen Pentensäureester mit Kohlenmonoxid und $C_1$- bis $C_4$-Alkanolen bei Temperaturen von 140 bis 200°C unter erhöhtem Druck zu Butandicarbonsäureestern umsetzt, wobei man

a) eine wäßrige Kobaltlösung bei Temperaturen von 50 bis 200°C und unter Drücken von 50 bis 500 bar mit überschüssigem Kohlenmonoxid und Wasserstoff in Gegenwart von Aktivkohle, die mit Kobaltcarbonyl beladen ist, behandelt,

d) die erhaltene wäßrige Lösung von Kobaltcarbonylwasserstoff mit Butadien oder Butadien enthaltenden Kohlenwasserstoffgemischen extrahiert und die wäßrige Phase abtrennt,

c) das Kobaltcarbonylhydrid, Kobaltcarbonyl und Butenylkobalttricarbonyl enthaltende Butadien oder Butadienkohlenwasserstoffgemisch mit Kohlenmonoxid und $C_1$- bis $C_4$-Alkanolen im Überschuß in Gegenwart von 0,5 bis 2 Mol tertiäre Stickstoffbasen je Mol Butadien mit einem $pK_a$-Wert von 3 bis 11 bei Temperaturen von 80 bis 150°C und unter Drücken von 300 bis 2 000 bar umsetzt,

d) aus dem erhaltenen Reaktionsgemisch die darin enthaltenen tertiären Stickstoffbasen bis auf 0,1 bis 0,3 Mol je Mol Pentensäureester sowie überschüssige Kohlenwasserstoffe abtrennt, den im Reaktionsgemisch verbleibenden Pentensäureester mit Kohlenmonoxid und $C_1$- bis $C_4$-Alkanolen im Überschuß bei Temperaturen von 140 bis 200°C und unter Drücken von 100 bis 400 bar in Gegenwart der im Reaktionsgemisch vorhandenen Menge an Kobaltcarbonyl und tertiären Stickstoffbasen umsetzt und anschließend überschüssige Alkanole und freie Stickstoffbasen abdestilliert und

e) daß verbleibende Kobaltkatalysatoren, Butandicarbonsäuren und Nebenprodukte enthaltende Reaktionsgemisch mit Oxidationsmitteln im wäßrig-sauren Medium behandelt und das Gemisch in eine organische Phase aus der durch Destillation Butandicarbonsäureester gewonnen werden und eine Kobaltsalze enthaltende wäßrige Phase trennt, *dadurch gekennzeichnet,* daß man die Kobaltsalze enthaltende wäßrige Phase mit Lösungsmitteln extrahiert, die mit Wasser nicht mischbar sind.

2. Verfahren nach Anspruch 1, *dadurch gekennzeichnet,* daß man die wäßrige Phase nach der Extraktion zusätzlich mit stark basischen Ionenaustauschern behandelt.

3. Verfahren nach Anspruch 1, *dadurch gekennzeichnet,* daß man die nach der Extraktion als organische Phase abgetrennten Lösungsmittel der organischen Phase in der Stufe e nach Behandeln mit Oxidationsmitteln in wäßrigsaurem Medium zufügt.

## Claims

1. A process for the manufacture of butanedicarboxylic acid esters, by reacting butadiene or a hydrocarbon mixture containing butadiene with carbon monoxide and an alkanol of 1 to 4 carbon atoms in the presence of a tertiary nitrogen base and a cobalt carbonyl compound at from 80 to 150° under superatmospheric pressure and then, without removing the catalyst, reacting the pentenoic acid ester obtained further with carbon monoxide and an alkanol of 1 to 4 carbon atoms at from 140 to 200°C under superatmospheric pressure, to give a butanedicarboxylic acid ester, wherein

a) an aqueous cobalt salt solution is treated, at from 50 to 200°C, under pressures of from 50 to 500 bar, with excess carbon monoxide and hydrogen in the presence of active charcoal charged with cobalt carbonyl,

b) the resulting aqueous solution of cobalt carbonyl hydride is extracted with butadiene or a hydrocarbon mixture containing butadiene and the aqueous phase is separated off,

c) the butadiene, or butadiene-containing hydrocarbon mixture, which contains cobalt carbonyl hydride, cobalt carbonyl and butenyl-cobalt tricarbonyl is reacted with carbon monoxide and an excess of an alkanol of 1 to 4 carbon atoms in the presence of from 0.5 to 2 moles, per mole of butadiene, of a tertiary nitrogen base having a $pK_a$ of from 3 to 11, at from 80 to 150°C, under a pressure of from 300 to 2,000 bar,

d) the tertiary nitrogen bases contained in the reaction mixture thus obtained are removed to the extent of leaving from 0.1 to 0.3 mole per mole of pentenoic acid ester produced, excess hydrocarbon is also removed, the pentenoic acid ester remaining in the reaction mixture is reacted with carbon monoxide and an excess of an alkanol of 1 to 4 carbon atoms at from 140 to 200°C under a pressure of from 100 to 400 bar in the presence of the amount of cobalt carbonyl and tertiary nitrogen base remaining in the reaction mixture and thereafter the excess alkanol and free tertiary nitrogen base are distilled off, and

e) the residual reaction mixture, containing cobalt catalyst, butanedicarboxylic acids and by-products, is treated with an oxidizing agent in an aqueous acid medium and the mixture is separated into an organic phase, from which butanedicarboxylic acid ester is isolated by distillation, and an aqueous phase containing cobalt salts, *characterized in that* the aqueous phase containing cobalt salts is extracted with a water-immiscible solvent.

2. A process as claimed in claim 1, *characterized in that* the aqueous phase, after the extraction, is additionally treated with a strongly basic ion exchanger.

3. A process as claimed in claim 1, *characterized in that* the solvent separated off as the organic phase after the extraction is added to the organic phase in stage e after treatment with an oxidizing agent in an aqueous acid medium.

## Revendications

1. Procédé de préparation d'esters d'acides butane-dicarboxyliques par réaction du butadiène ou d'un mélange d'hydrocarbures comprenant du butadiène entre 80 et 150°C sous pression accrue avec l'oxyde de carbone et des alcanols en $C_1$ à $C_4$ en présence de bases azotées tertiaires et de cobalt-carbonyles, suivie de la transformation de l'ester d'acide penténique formé en esters d'acides butane-dicarboxyliques par réaction entre 140 et 200°C sous pression accrue avec 1'oxyde de carbone et les alcanols en $C_1$ à $C_4$, comportant:

a) le traitement d'une solution aqueuse de cobalt entre 50 et 200°C sous une pression de 50 à 500 bars et en présence de charbon activé, chargé de cobalt-carbonyle, avec de l'hydrogène et de l'oxyde de carbone en excès; à $C_4$ en excès en présence du cobalt-carbonyle et des bases azotées tertiaires rériduelles contenus dans le mélange réactionnel, puis de l'élimination par distillation des bases azotées libres et des alcanols en exces;

b) l'extraction de la solution aqueuse de cobalt-carbonyl-hydrogène ainsi obtenue par le butadiène ou un mélange d'hydrocarbures comprenant du butadiène, suivie de la séparation de la phase aqueuse;

c) la réaction du butadiène ou du mélange d'hydrocarbures comprenant du butadiène, contenant de l'hydrure de cobalt-carbonyle, du cobalt-carbonyle et due buténylcobalt-tricarbonyle, entre 80 et 150°C sous une pression de 300 à 2000 bars avec l'oxyde de carbone et les alcanols en $C_1$ à $C_4$ en excès en présence, par mole de butadiène, de 0,5 à 2 moles de bases azotées tertiaires avec un $pK_a$ de 3 à 11;

d) l'extraction du mélange réactionnel obtenu des bases azotées tertiaires jusqu'à une proportion résiduelle de 0,1 à 0,3 mole par mole d'ester d'acide penténique et des hydrocarbures en excès, suivie de la réaction de l'ester d'acide penténique subsistant dans le mélange réactionnel entre 140 et 200°C sous une pression de 100 à 400 bars avec l'oxyde de carbone et les alcanols en $C_1$ à $C_4$ en excès en présence du cobalt-carbonyle et des bases azotées tertiaires résiduelles contenus dans le mélange réactionnel, puis de l'élimination par distillation des bases azotées libres et des alcanols en excès;

e) le traitement due mélange réactionnel résiduel, contenant les catalyseurs au cobalt, des acides butane-dicarboxyliques et des produits secondaires, dans son milieu auqueuxacide par un oxydant, suivi de la séparation d'une phase organique, de laquelle les esters d'acides butane-dicarboxyliques sont extraits par distillation, et d'une phase aqueuse contenant des sels du cobalt,

caractérisé en ce que l'extraction de la phase aqueuse contenant des sels du cobalt est effectuée à l'aide de solvants non miscibles à l'eau.

2. Procédé suivant la revendication 1, caractérisé en ce que l'extraction de la phase aqueuse par un solvant non miscible à l'eau est suivie d'un traitement de la phase aqueuse avec un échangeur d'ions fortement basique.

3. Procédé suivant la revendication 1, caractérisé en ce que le solvant d'extraction de la phase aqueuse, séparé comme phase organique, est ajouté à la phase organique séparée dans le stade opératoire *e* après le traitement du milieu aqueux acide par un oxydant.